# EUROPEAN PATENT SPECIFICATION

(11) **EP 1 863 464 B1**
(45) Date of publication and mention of the grant of the patent: **11.11.2009**
(21) Application number: 06739215.9
(22) Date of filing: 22.03.2006
(51) Int. Cl.: A61K 31/137, C07D 209/12, C07C 215/64, A61K 31/404, A61K 31/55, A61P 25/00, A61P 5/24

(54) **O-DESMETHYLVENLAFAXINE AND BAZEDOXIFENE COMBINATION PRODUCT AND USES THEREOF**
KOMBINATIONSPRODUKT AUS O-DESMETHYLVENLAFAXIN UND BAZEDOXIFEN UND VERWENDUNGEN DAVON
PRODUIT COMBINE A BASE D'O-DESMETHYLVENLAFAXINE ET DE BAZEDOXIFENE ET UTILISATIONS DE CE PRODUIT

(30) Priority: 31.03.2005 US 666902 P
(43) Date of publication of application: 12.12.2007
(73) Proprietor: Wyeth, Madison, NJ 07940 (US)
(72) Inventor: SHAH, Syed, East Hanover, New Jersey 07936 (US); FAWZI, Mahdi, Morristown, New Jersey 07960 (US); DIORIO, Christopher, Campbell Hall, New York 10916 (US)
(74) Representative: Talbott, Dawn Jacqueline
(86) International application number: PCT/US2006/010335
(87) International publication number: WO 2006/104791

(56) References cited:
- WO-A-02/03975
- WO-A-02/064543
- WO-A-03/105834
- MUTH E A ET AL: "BIOCHEMICAL, NEUROPHYSIOLOGICAL, AND BEHAVIORAL EFFECTS OF WY-45,233 AND OTHER IDENTIFIED METABOLITES OF THE ANTIDEPRESSANT VENLAFAXINE" DRUG DEVELOPMENT RESEARCH, NEW YORK, NY, US, vol. 23, no. 2, 1991, pages 191-199, XP000923124 ISSN: 0272-4391

## Description

### BACKGROUND OF THE INVENTION

This invention relates generally to the field of estrogen receptor modulators, and to treatment of conditions associated with low estrogen expression and/or low estrogen receptor expression.

O-desmethylvenlafaxine (ODV), the major metabolite of venlafaxine, selectively blocks the reuptake of serotonin and norepinephrine. Klamerus, K. J. et al., "Introduction of the Composite Parameter to the Pharmacokinetics of Venlafaxine and its Active O-Desmethyl Metabolite", J. Clip. Pharmacol. 32:716-724 (1992). O-desmethylvenlafaxine, chemically named 1-[2-(dimethylamino)-1-(4-phenol)ethyl]-cyclohexanol, was exemplified as a fumarate salt in US Patent No. 4,535,186. However, the fumarate salt of O-desmethylvenlafaxine has unsuitable physicochemical and permeability characteristics. O-desmethylvenlafaxine is also exemplified as a free base in International Patent Publication No. WO 00/32555.

The succinate form of ODV has been described [US Patent No. 6,673,838]. The succinate monohydrate form of ODV has been incorporated into an extended release hydro-gel tablet, which reduces adverse effects such as nausea, vomiting, diarrhea, and abdominal pain. Formulations describing the use of hydroxypropyl methylcellulose (HPMC) as the hydrogel matrix have been described [International Patent Publication No. WO 02/064543 A2].

Bazedoxifene acetate (1-[4-(2-azepan-1-yl-ethoxy)-benzyl]-2-(4-hydroxy phenyl)-3-methyl-1H-indol-5-ol acetic acid), having the chemical formula shown below: 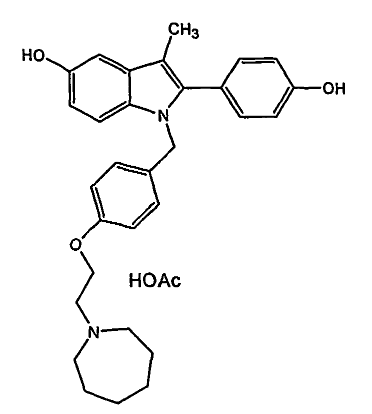 belongs to the class of drugs typically referred to as selective estrogen receptor modulators (SERMs). Consistent with its classification, bazedoxifene demonstrates affinity for estrogen receptors (ER) but shows tissue selective estrogenic effects. For example, bazedoxifene acetate demonstrates little or no stimulation of uterine response in preclinical models of uterine stimulation. Conversely, bazedoxifene acetate demonstrates an estrogen agonist-like effect in preventing bone loss and reducing cholesterol in an ovariectomized rat model of osteopenia. In an MCF-7 cell line (human breast cancer cell line), bazedoxifene acetate behaves as an estrogen antagonist. These data demonstrate that bazedoxifene acetate is estrogenic on bone and cardiovascular lipid parameters and antiestrogenic on uterine and mammary tissue and thus has the potential for treating a number of different disease or disease-like states wherein the estrogen receptor is involved.

US Patent Nos. 5,998,402 and 6,479,535 report the preparation of bazedoxifene acetate. The synthetic preparation of bazedoxifene acetate has also appeared in the general literature. See, for example, Miller, et al., J. Med. Chem., 2001, 44, 1654-1657. Further description of the drug's biological activity has appeared in the general literature as well (*e.g*., Miller, et al., Drugs of the Future, 2002, 27(2), 117-121). Formulations of bazedoxifene acetate are also reported in US Patent Application Publication No. 2002/0031548 A1.

What are needed are improved methods of treating conditions associated with low estrogen expression and/or low estrogen receptor expression.

### SUMMARY OF THE INVENTION

In one aspect, the invention provides an orally administrable combination product comprising as active compounds O-desmethylvenlafaxine succinate (DVS) in a sustained release formulation and bazedoxifene or a pharmaceutically acceptable salt thereof in an immediate release formulation.

In another aspect, the invention provides a multi-layer tablet or capsule in which each active compound is found in a separate layer. In one embodiment, one or both layers is a compressed granulation. In another embodiment, one of the layers is a solid dispersion blend. In still another embodiment, the invention provides a capsule containing a multiparticulate and a granulation. In a further embodiment, the core is composed of one active compound and another active compound is provided in a coating layer.

In another aspect, the invention provides the use of a composition of the invention for preparing a medicament.

Still other aspects and advantages of the invention will be apparent from the following detailed description of the invention.

### DETAILED DESCRIPTION OF THE INVENTION

The present invention provides an orally administrable combination products useful for treating disease states or syndromes associated with an estrogen deficiency or an excess of estrogen, as well as in the preparation of medicaments useful therefor. The compositions of the invention are also useful in methods of treatment for diseases or disorders which result from proliferation or abnormal development, actions or growth of endometrial or endometrial-like tissues, as well as in the preparation of medicaments useful therefor.

The combination of the invention utilized as active ingredients, at a minimum, O-desmethylvenlafaxine succinate and bazedoxifene or a pharmaceutically acceptable salt thereof. In one embodiment, the active ingredients are formulated into a single unit dose combination product, *e.g*., a tablet, capsule, or caplet.

As used herein, O-desmethylvenlafaxine or ODV refers to 1-[2-(dimethylamino)-1-(4-phenol)ethyl]-cyclohexanol. Several pharmaceutically acceptable salts thereof have been described, including, e.g., the fumarate salt thereof [US Patent No. 4,535,186], the succinate salt form of ODV [US Patent No. 6,673,838], among others. ODV is also exemplified as a free base in International Patent Publication No. WO 00/32555.

The succinate salt of ODV (DVS or desvenlafaxine succinate) can be prepared as described in US Patent No. 6,673,838.

As used herein, bazedoxifene acetate refers to 1-[4-(2-azepan-1-yl-ethoxy)-benzyl]-2-(4-hydroxy-phenyl)-3-methyl-1H-indol-5-ol acetic acid. US Patent Nos. 5,998,402 and 6,479,535 report the preparation of bazedoxifene and salts thereof. The synthetic preparation of bazedoxifene acetate (BZA) has also appeared in the general literature. See, for example, Miller, et al., J. Med. Chem., 2001, 44, 1654-1657. Alternatively, one of skill in the art can substitute other salts of bazedoxifene for the BZA described in the examples herein.

The terms "pharmaceutically acceptable salts" and "pharmaceutically acceptable salt" refer to salts derived from organic and inorganic acids such as, for example, acetic, lactic, citric, cinnamic, tartaric, succinic, fumaric, maleic, malonic, mandelic, malic, oxalic, propionic, hydrochloric, hydrobromic, phosphoric, nitric, sulfuric, glycolic, pyruvic, methanesulfonic, ethanesulfonic, toluenesulfonic, salicylic, benzoic, and similarly known acceptable acids.

In one aspect, the invention provides an orally administrable product containing the two active ingredients. The product is designed to contain bazedoxifene in an immediate release formulation and O-desmethylvenlafaxine in a slow release formulation.

These two active ingredients may be formulated separately, but in the same form, e.g., each may be in a granulation. Alternatively, the product may contain the active compounds in different forms, *e.g*., a granulation and a multiparticulate, a granulation and a solid dispersion blend, a multiparticulate and a solid dispersion blend, or in other forms as may be desired.

In one embodiment, the tablet is a multi-layer tablet, in which the active components are contained in separate layers. In one embodiment, the tablet contains two layers with active compounds. In another aspect, the invention provides a capsule. Such a capsule may be filled with a tablet as described herein, or contain the two actives in different formulations. In one embodiment, a capsule according to the invention contains desvenlafaxine succinate multiparticulates and bazedoxifene or a pharmaceutically acceptable salt thereof. In a further embodiment, the bazedoxifene or pharmaceutical salt thereof is in the form of a solid dispersion. In another embodiment, the bazedoxifene or pharmaceutically acceptable salt thereof is in the form of a granulation. In yet another embodiment, the bazedoxifene is a coating applied to the multiparticulates. In a further embodiment, a pharmaceutically acceptable salt of bazedoxifene is used. In another embodiment, a multiparticulate contains desvenlafaxine succinate and microcrystalline cellulose.

In a further embodiment, the bazedoxifene is provided as a coating over a DVS layer, DVS tablet, or DVS multiparticulate core. In the examples below, a bazedoxifene film coat over a DVS tablet core and a bazedoxifene, sugar-based, coat over a DVS tablet core are illustrated.

In one embodiment, a tablet according to the invention is prepared having a first layer containing desvenlafaxine succinate, hypomellose, microcrystalline cellulose, talc, and magnesium stearate, and a second layer containing bazedoxifene or a pharmaceutically acceptable salt thereof. In one embodiment, the ODV formulation of the first layer contains hypomellose, microcrystalline cellulose, and talc. In a further embodiment, the DVS layer is composed of:

| | |
|---|---|
| desvenlafaxine succinate | 45- 55 wt% |
| hypomellose | 35 - 45 wt % |
| microcrystalline cellulose | 3 - 4 wt % |
| talc | 4 - 5 wt% |
| magnesium stearate | 1 - 2 wt%. |

In one embodiment, the DVS is about 50 wt% and the hypomellose is about 40 wt % of that layer. Suitably, this mixture can be prepared in the form of a granulation or another suitable form.

In another embodiment, the second contains bazedoxifene acetate, lactose, microcrystalline cellulose, and starch. Additional BZA formulations are described below.

Regardless of the form of the combination product, the product desirably contains as a unit dose, about 10 mg to 500 mg O-desmethylvenlafaxine succinate (DVS), wherein the dose amount is calculated based on the amount of O-desmethylvenlafaxine free base, and about 5 mg to 100 mg bazedoxifene or a pharmaceutically acceptable salt thereof, calculated based on the amount of bazedoxifene.

In another embodiment, the product contains bazedoxifene acetate (BZA). In yet another embodiment, the bazedoxifene is BZA. However, the invention is not so limited.

In other embodiments, the unit dose is in the range of 25 mg to 250 mg DVS, or 50 mg to 200 mg DVS, or about 150 mg DVS, as calculated based on the amount of O-desmethylvenlafaxine free base. In these and other embodiments, the unit dose of bazedoxifene is in the range of 10 mg to 75 mg bazedoxifene, 20 mg to 50 mg bazedoxifene, about 25 mg to about 40 mg bazedoxifene, or about 20 mg bazedoxifene, calculated on the basis of free bazedoxifene.

Suitably, the ODV succinate salt is admixed with one or more components selected from the group including, diluents, binders, fillers, glidants, anti-adherents, and adjuvants. The binder may be selected from among known binders, including, e.g., cellulose, and povidone, among others. In one embodiment, the binder is selected from among microcrystalline cellulose, crospovidone, and mixtures thereof.

Suitable pH adjusters include, *e.g*., sodium carbonate, sodium bicarbonate, potassium carbonate, lithium carbonate, among others. Still other suitable components will be readily apparent to one of skill in the art.

In one embodiment, the DVS in a formulation which contains rate-controlling components. Typically, such rate controlling components are rate controlling polymers selected from among hydrophilic polymers and inert plasticized polymers. Suitable rate controlling hydrophilic polymers include, without limitation, polyvinyl alcohol (PVA), hypomellose and mixtures thereof. Examples of suitable insoluble or inert "plastic" polymers include, without limitation, one or more polymethacrylates (*i.e*., Eudragit® polymer). Other suitable rate-controlling polymer materials include, *e.g*., hydroxyalkyl celluloses, poly(ethylene) oxides, alkyl celluloses, carboxymethyl celluloses, hydrophilic cellulose derivatives, and polyethylene glycol.

In one embodiment, an DVS multiparticulate can be prepared. See, *e.g.,* US Patent Application Publication No. US 2005/0175698 A1 (published August 11, 2005), entitled, "Multiparticulate O-Desmethylvenlafaxine Salts and Uses Thereof". In a further embodiment, when the multiparticulate DVS is a spheroid, bead or pellet, the multiparticulate is in the range of about 0.6 mm to about 1 mm in size. However, the multiparticulate may vary in size, without departing from the present invention.

The multiparticulate DVS of the invention are composed, at a minimum, of a core composed of DVS, and one or more diluents, binders, fillers, glidants, anti-adherents, a pH adjuster and/or an adjuvant.

Suitably, the total amount of diluent, binders, fillers, glidants, anti-adherents, and adjuvants present in the core is an amount of about 30% w/w to about 97% w/w of the multiparticulate core. For example, when present, a binder, diluent and/or filler can each be present in an amount of about 15% w/w to about 80% w/w, or about 20% w/w to about 70% w/w, or about 25% w/w to about 45% w/w, or about 30% w/w to about 42% w/w of the uncoated dosage form. The total amount of a pH adjuster in the formulation can range from about 0.1 % w/w to about 10% w/w of the core, or about 1% w/w to about 8% w/w, or about 3% w/w to about 7% w/w. However, these percentages can be adjusted as needed or desired by one of skill in the art.

The binder may be selected from among known binders, including, *e.g.,* cellulose, and povidone, among others. In one embodiment, the binder is selected from among microcrystalline cellulose, crospovidone, and mixtures thereof.

Suitable pH adjusters include, *e.g.,* sodium carbonate, sodium bicarbonate, potassium carbonate, lithium carbonate, among others. Still other suitable components will be readily apparent to one of skill in the art.

In one embodiment, the DVS is in a sustained release formulation which contains rate-controlling components. In a further embodiment, such rate controlling components are rate controlling polymers selected from among hydrophilic polymers and inert plasticized polymers. Suitable rate controlling hydrophilic polymers include, without limitation, polyvinyl alcohol (PVA), hypomellose and mixtures thereof. Examples of suitable insoluble or inert "plastic" polymers include, without limitation, one or more polymethacrylates (*i.e.,* Eudragit® polymer). Other suitable rate-controlling polymer materials include, *e.g.,* hydroxyalkyl celluloses, poly(ethylene) oxides, alkyl celluloses, carboxymethyl celluloses, hydrophilic cellulose derivatives, and polyethylene glycol.

In one embodiment, an DVS multiparticulate of the invention contains about 5% w/w to about 75% w/w microcrystalline cellulose (MCC), about 10% w/w to about 70% w/w MCC, about 20% w/w to about 60% w/w, or about 30% w/w to about 50% w/w, based on the weight of the uncoated multiparticulate. In one desirable embodiment, the ODV multiparticulate core contains about 70 wt% to about 30 wt% based on the uncoated multiparticulate.

In one embodiment, the multiparticulate DVS core is uncoated. The multiparticulates can be placed into a suitable capsule shell or compressed into tablets, using techniques know to those of skill in the art. Suitably, the resulting capsule shell or compressed tablets contain 10 mg to 400 mg of ODV.

In other embodiments, the multiparticulate DVS contain one or more coatings over the core. In still other embodiments, the multiparticulate consists of a pellet core and non-functional seal coating and a functional second coating.

In one embodiment, an initial seal coat can be applied directly to the core. Although the components of this seal coat can be modified by one of skill in the art, the seal coat may be selected from among suitable polymers such as hydroxypropyl methylcellulose (HPMC), ethylcellulose, polyvinyl alcohol, and combinations thereof, optionally containing plasticizers and other desirable components. In a further embodiment, the seal coat contains HPMC. In one embodiment, a seal coat is applied as a HPMC solution at a concentration of about 3% w/w to about 25% w/w, or about 5% w/w to about 7.5% w/w. Upon drying, under suitable conditions, the initial seal coat is in the range of about 1 % w/w to about 3% w/w, or about 2% w/w, of the uncoated multiparticulate. In another embodiment, a commercially available seal coat containing HPMC, among other inert components, is utilized. One such commercially available seal coat is Opadry® Clear (Colorcon, Inc.).

In one embodiment, the multiparticulates can contain a further coating layer over the initial seal coat, if present, or directly to the uncoated multiparticulate DVS core, to provide a delay release formulation. These formulations may also lower the incidence of the side effects, including nausea, emesis, and irritable bowel syndrome. Without wishing to be bound by theory, it is believed that these side-effects are avoided by by-passing release in the upper GI tract and providing release in the lower GI tract.

An enteric coat (rate-controlling film) may be applied to the multiparticulates and may include, but is not limited to polymethacrylates, hypomellose, ethylcellulose, or a combination thereof. The modified release multiparticulate formulation can contain from about 3% w/w to about 70% w/w of DVS and from about 5% w/w to about 75% w/w microcrystalline cellulose, based on the weight of an uncoated multiparticulate.

In one embodiment, the enteric coat contains a product which is a copolymer of methacrylic acid and methacrylates, such as the commercially available Eudragit® L 30 K55 (Röhm GmbH & Co. KG). In a further embodiment, the enteric coat is applied such that it coats the multiparticulate in an amount of about 15 to about 45% w/w, about 20% w/w to about 30% w/w, or about 25% w/w to about 30% w/w of the uncoated or initially-coated multiparticulate. In one embodiment, the enteric coat is composed of a Eudragit® L30D-55 copolymer (Röhm GmbH & Co. KG), talc, triethyl citrate, and water. In still another embodiment, the enteric coating contains about 30% w/w of a 30 wt% dispersion of Eudragit® L 30 D55 coating; about 15% w/w talc, about 3% triethyl citrate; a pH adjuster such as sodium hydroxide; and water.

In another embodiment, the enteric coat contains an ethylcellulose-based product, such as the commercially available Surelease® aqueous ethylcellulose dispersion (25% solids) product (Colorcon, Inc.). In one embodiment, a solution of Surelease® dispersion of about 3% w/w to about 25% w/w, or about 3% to about 7%, or about 5% w/w, is applied to the multiparticulate. Upon drying under suitable conditions, the enteric coat is in the range of about 2% to about 5%, or about 3% to about 4% w/w of the uncoated or initially-coated multiparticulate.

Other formulations containing DVS, can be determined by one of skill in the art.

### BZA Formulations

In another aspect, a composition of the invention contains as an active ingredient, bazedoxifene or a pharmaceutically acceptable salt thereof, containing 5 mg to 100 mg bazedoxifene. The bazedoxifene formulation can form a separate layer from the DVS in a tablet, or other combination product.

In one embodiment, the bazedoxifene formulation contains bazedoxifene acetate, lactose, microcrystalline cellulose, and starch.

In another embodiment, the bazedoxifene formulation contains:

| | |
|---|---|
| bazedoxifene acetate | 5 - 40 wt% |
| lactose | 30 - 35 wt% |
| microcrystalline cellulose | 25 - 30 wt% |
| pregelatized starch | 12 -18 wt% |
| sodium lauryl sulfate | 1- 2 wt% |
| sodium starch glycolate | 5 - 8 wt% |
| ascorbic acid | 1-2 wt% |
| silicon dioxide | < 1 wt% |
| magnesium stearate | < 1 wt%. |

In yet another embodiment, the bazedoxifene formulation contains:

| | |
|---|---|
| bazedoxifene acetate | 10 wt% |
| lactose | 30 - 35 wt% |
| microcrystalline cellulose | 25 - 30 wt% |
| pregelatized starch | 12 -16 wt% |
| sodium lauryl sulfate | 1- 2 wt% |
| sodium starch glycolate | 5 - 8 wt% |
| ascorbic acid | 1- 2 wt% |
| silicon dioxide | < 1 wt% |
| magnesium stearate | < 1 wt%. |

These formulations are particularly well suited for the preparation of a granulation. In one embodiment, the product contains a 40 mg granulation ofbazedoxifene acetate.

### BZA Solid Dispersion Blend

In one embodiment, the product contains a layer having a solid dispersion blend having an amount of bazedoxifene or a salt thereof equivalent to 5 mg to 100 mg, about 10 mg to 50 mg, about 25 mg to about 40 mg bazedoxifene. In another embodiment, the solid dispersion blend contains about 20 mg bazedoxifene or a salt thereof.

In one embodiment, the compositions of the invention contain BZA dispersed in a dispersing agent. In another embodiment, the weight ratio of BZA to dispersing agent is about 1:99 to about 99:1. In yet another embodiment, the weight ratio of BZA to dispersing agent is about 1:99 to about 75:25 or about 1:99 to about 60:40. In a further embodiment, the weight ratio of BZA to dispersing agent is about 1:99 to about 15:85; about 1:99 to about 10:90; or about I:99 to about 5:95. In another embodiment, the weight ratio of BZA to dispersing agent is about 5:95. In still another embodiment, the weight ratio of BZA to dispersing agent is about 25:75 to about 75:25, about 40:60 to about 60:40, or about 1:1. In another embodiment, the weight ratio of BZA to dispersing agent is about 1:1.

The "dispersing agent," as used herein, refers to any substance or mixture of substances that acts as a suspending medium for particles of solid bazedoxifene acetate. The dispersing agent is typically composed of a pharmaceutically acceptable substance that does not substantially interfere with the pharmaceutical action of BZA. The phrase "pharmaceutically acceptable" is employed herein to refer to those substances which are, within the scope of sound medical judgment, suitable for use in contact with the tissues of human beings and animals without excessive toxicity, irritation, allergic response, or other problem or complication, commensurate with a reasonable benefit/risk ratio. In some embodiments, the dispersing agent is a solid at room temperature (*e.g.,* about 22°C). In further embodiments, the dispersing agent melts at a temperature between about 30°C and 100°C. In further embodiments, the dispersing agent is soluble in an organic solvent.

Non-limiting examples of suitable dispersing agents include polymers such as celluloses (*e.g.,* carboxymethylcelluloses, methylcelluloses, hydroxypropylcelluloses, hydroxypropylmethylcelluloses); hyaluronates; alginates; polysaccharides, heteropolysaccharides (pectins); poloxamers; poloxamines; ethylene vinyl acetates; polyethylene glycols; dextrans; polyvinylpyrrolidones; chitosans; polyvinylalcohols; propylene glycols; polyvinylacetates; phosphatidylcholines (lecithins); miglyols; polylactic acid; polyhydroxybutyric acid; mixtures of two or more thereof, copolymers thereof, derivatives thereof, and the like. Further examplary dispersing agents include copolymer systems such as polyethylene glycol-polylactic acid (PEG-PLA), polyethylene glycol-polyhydroxybutyric acid (PEG-PHB), polyvinylpyrrolidone-polyvinylalcohol (PVP-PVA), and derivatized copolymers such as copolymers of N-vinyl purine (or pyrimidine) derivatives and N-vinylpyrrolidone.

In one embodiment, the dispersing agent contains polyvinylpyrrolidone (PVP) or a derivative thereof. PVP is a polyamide that forms complexes with a wide variety of substances and is considered to be chemically and physiologically inert. Examples of suitable PVPs include polyvinylpyrrolidone(s) having an average molecular weight from about 10,000 to about 50,000. In one embodiment, the polyvinylpyrrolidone has an average molecular weight of about 10,000 to about 20,000. In another embodiment, the polyvinylpyrrolidone has a molecular weight of about 15,000 to about 20,000. In yet another embodiment, multiple polyvinylpyrrolidones are utilized in the dispersing agent. In still another embodiment, polyvinylpyrrolidones of differing weights may be utilized. An example suitable PVP is PVP K-17 (PLASDONE povidone, ISP Technologies, Ltd.). In one embodiment, the dispersing agent consists essentially of PVP or derivative thereof.

In one embodiment, the dispersing agent contains a block co-polymer of ethylene and propylene glycol, often referred to as a Poloxamer. Some suitable exemplary Poloxamers include Poloxamer 188 (LUTROL F 68, BASF), Poloxamer 407 (LUTROL F 127, BASF), and the like. In a further embodiment, the dispersing agent is Poloxamer 188.

In one embodiment, the dispersing agent contains a polyethylene glycol (PEG). Suitable PEGs include PEG 200, 300, 400, 600, 1000, 1450, 3350, 4000, 6000, 8000, 10000, 20000, mixtures thereof and the like. In a further embodiment, the dispersing agent is PEG 1450.

The BZA dispersions useful in the invention can be made by any method that results in, for example, a solid dispersion of amorphous BZA. In one embodiment, BZA (in any form, *e.g.,* crystalline, amorphous, *etc.*) and the dispersing agent are dissolved in a dispersing solvent (together, or separately and then combined) in the weight ratio desired and then the dispersing solvent is removed to yield the desired solid dispersion. The dispersing solvent can be an aqueous solvent or organic solvent. Suitable organic solvents include alcohols, ethers, hydrocarbons, halogenated hydrocarbons, nitriles, mixtures thereof, and the like. In another embodiment, the organic solvent is a volatile solvent such as methanol, ethanol, isopropanol, diethyl ether, pentane, hexane, benzene, dichloromethane, acetonitrile, mixtures thereof and the like. In a further embodiment, the organic solvent is an alcohol such as methanol, ethanol, n-propanol, ispropanol, mixtures thereof and the like. In some embodiments, the organic solvent is ethanol.

In one embodiment, BZA and dispersing agent are combined in the desired weight ratio when either or both the BZA and dispersing agent is (are) in liquid form (*e.g.,* a melt), and then the liquid mixture is solidified to form the desired solid dispersion. In a further embodiment, the BZA and dispersing agent are combined when at least one of the BZA and dispersing agent is melted. The resulting mixture is then solidified by cooling to a temperature sufficient to solidify the mixture. In a further embodiment, the mixture is cooled to about 25 °C or below. In another embodiment, BZA is combined with melted dispersing agent and the resulting mixture cooled to a temperature below the melting point of the mixture to form the solid dispersion. In one embodiment, the dispersing agent is heated to a temperature between about 30 and 200°C, between about 30 and 150°C, or between about 30 and 100°C, which is a temperature that is at or above the melting point of the dispersing agent. In a further embodiment, the dispersing agent is heated to a temperature above about 30°C, above about 40°C, above about 50°C, above about 60°C, above about 70°C, above about 80°C, or above about 90°C. These and other methods are routine techniques suitable for the preparation of the BZA dispersions of the invention.

In one embodiment, the solid dispersions useful in the invention are characterized by an equilibrium solubility in 0.0005 M acetic acid at a temperature of about 20 to about 26°C that is greater than that for crystalline or microcrystalline bazedoxifene acetate. In a further embodiment, the solid dispersions of the invention are characterized by an equilibrium solubility in 0.0005 M acetic acid at a temperature of about 20 to about 26 °C that is at least about 8, at least about 10, at least about 12, at least about 14, at least about 16, or at least about 19 mg/mL. Equilibrium solubility can be measured by routine methods in the art.

In one embodiment, the solid dispersions useful in the invention are characterized such that a dosage form comprising about 10 mg total of bazedoxifene acetate in a solid dispersion is characterized by an AUC₀₋₂₄ greater than about 140, greater than about 150, greater than about 160, greater than about 170, or greater than about 180 ng·hr/mL when orally administered to mammal. In a further embodiment, the solid dispersions useful in the invention are characterized such that a dosage form comprising about 10 mg total of bazedoxifene acetate in a solid dispersion is characterized by: a) an AUC₀₋₂₄ of about 140 to about 250 ng·hr/mL; b) a Cₘₐₓ of about 12 to about 30 ng/mL; and c) a tₘₐₓ of about 1.0 to about 3.5 hr; when orally administered to mammal. Methods for measuring the pharmacokinetic parameters AUC₀₋₂₄ (area under curve for 24 hours), Cₘₐₓ, and tₘₐₓ are well known and described in the art.

Additional details on preparation and characterized of the solid BZA dispersion useful in the present invention are described in US Patent Publication No. US 2005/0227966 A1, published October 13, 2005.

In one embodiment, a product of the invention contains a layer of a solid dispersion blend of BZA having the formulation:

| | |
|---|---|
| bazedoxifene:polyvinylpyrorridone solid dispersion | about 40 wt% |
| microcrystalline cellulose | about 50 wt% |
| croscarmellose sodium | about 10 wt%. |

In another embodiment, the formulation contains about 1 wt% magnesium stearate, or less. Still other bazedoxifene granulations or solid dispersion blends, or other formulations can be utilized.

### BZA Coating Layers

In one embodiment, the bazedoxifene is provided in the form of a coat or a layer applied over a DVS core (*e.g.,* a layer, a multiparticulate, or a tablet). The coat may be applied directly over the DVS core or there may be intermediate layers.

In one embodiment, the bazedoxifene coat provides an amount of 4 - 6 wt% bazedoxifene acetate, and preferably about 5 wt% bazedoxifene per dosage unit. In another embodiment, bazedoxifene is provided in a coating layer in an amount of about 20 mg per tablet.

Bazedoxifene can be mixed into a suitable coating suspension and applied using conventional spray methods. In one embodiment, BZA is mixed into a hypomellose-based clear coat system (e.g., Opadry® clear coat) at a ratio of about 1:2 BZA to Opadry® clear coat (based on weight). In another embodiment, BZA is mixed into a sugar based overcoat. In still another embodiment, BZA is mixed with a sugar-based coating suspension containing sucrose, hypomellose, sucrose palmitate and ascorbic acid.

Still other suitable coating solutions will be apparent to one of skill in the art.

### I. Method of Producing Formulations of Invention

In one aspect, the present invention provides a method of preparing a combination product from separately formulated DVS thereof and a bazedoxifene combination product.

In one embodiment, the active components are prepared in the form of a multi-layer tablet or tablet-in-capsule. For example, a O-desmethylvenlafaxine succinate is mixed with suitable excipients to form a first granulation and a bazedoxifene is mixed with suitable excipients to form a second granulation. In one embodiment, one or both granulations are formed using a roller compactor. In another embodiment, one or both granulations are formed using a high shear granulator. However, other methods known to those of skill in the art, including, *e.g.,* a low shear granulator, a blender, *etc.*, can be utilized to prepare suitable granulations. The first granulation and the second granulation are then compressed using conventional methods to form a bi-layer tablet. This tablet may be provided with additional layers, optionally, containing additional layers with active components, or other layers as may be desired for enteric coating, seal coating, separation between layers, or the like. In one embodiment, the tablet core contains only one of the active components and the other active component is provided in a coating layer.

In another embodiment, a final seal coat is applied over the tablet. Suitably, this final seal coat is composed of hydroxypropyhmethylcellulose (HPMC) and water, upon drying, is less than about 1 wt% of the total, coated tablet. In a further embodiment, talc is utilized as a final step prior to filling the multi-layer tablets into a suitable packaging unit.

Alternatively or additional, the tablet may be loaded into a capsule.

In another aspect, the invention provides a capsule containing the separately formulated active components in different forms. For example, the capsule may contain a granulation of one active and a multiparticulate of the other active; a multiparticulate of one active and a solid dispersion of the other active; a granulation of one active and a solid dispersion of the other active; a core containing one active compound with the other active components in a coating layer. Such capsules are produced using techniques known to those of skill in the art.

Also encompassed by the invention are pharmaceutical packs and kits comprising a container, such as a foil package or other suitable container, having a formulation of the invention in unit dosage form.

### II. Use of Combination Products of the Invention

The compositions of the invention are useful for treating, or for the preparation of medicaments useful in the treatment of, many maladies which result from estrogen effects and estrogen excess or deficiency (*e.g.,* low circulating levels of estrogen) including osteoporosis, prostatic hypertrophy, male pattern baldness, vaginal and skin atrophy, acne, dysfunctional uterine bleeding, endometrial polyps, benign breast disease, uterine leiomyomas, adenomyosis, ovarian cancer, infertility, breast cancer, endometriosis, endometrial cancer, polycystic ovary syndrome, cardiovascular disease, contraception, Alzheimer's disease, cognitive decline and other CNS disorders, as well as certain cancers including melanoma, prostrate cancer, cancers of the colon, CNS cancers, among others. Additionally, the compositions can be used for contraception in pre-menopausal women, as well as hormone replacement therapy in post-menopausal women (such as for treating vasomotor disturbances such as hot flush) or in other estrogen deficiency states where estrogen supplementation would be beneficial. It can also be used in disease states where amenorrhea is advantageous, such as leukemia, endometrial ablations, chronic renal or hepatic disease or coagulation diseases or disorders.

The compositions of the invention can also be used for inhibition of, or for the preparation of medicaments useful in the inhibition of, bone loss, which can result from an imbalance in a subject's formation of new bone tissues and the resorption of older tissues, leading to a net loss of bone. Such bone depletion results in a range of individuals, particularly in post-menopausal women, women who have undergone bilateral oophorectomy, those receiving or who have received extended corticosteroid therapies, those experiencing gonadal dysgenesis, and those suffering from Cushing's syndrome. Special needs for bone (including teeth and oral bone) and bone replacement can also be addressed using the present solid dispersion in individuals with bone fractures, defective bone structures, and those receiving bone-related surgeries and/or the implantation of prosthesis. In addition to the problems described above, the compositions and methods of the invention can be used in treatments for, or in the preparation of medicaments useful in treating, osteoarthritis, hypocalcemia, hypercalcemia, Paget's disease, osteomalacia, osteohalisteresis, multiple myeloma and other forms of cancer having deleterious effects on bone tissues.

In addition, the combination of the invention is useful in alleviating the symptoms of depression, fibromyalgia, anxiety, stress urinary incontinence (S.U.I.), irritable bowel syndrome (I.B.S.), neuropathic pain, and other post meno-pausal symptoms including, *e.g.,* host flushes, osteoporosis, vaginal atrophy, *etc*.

As used herein, the term "treating" in reference to a disease is meant to refer to preventing, inhibiting and/or ameliorating the disease.

As used herein, the term "subject", "individual" or "patient," used interchangeably, refers to any mammals, preferably mice, rats, other rodents, rabbits, dogs, cats, swine, cattle, sheep, horses, or primates, and most preferably humans.

As used herein, the phrase "therapeutically effective amount" refers to the amount of active compound or pharmaceutical agent that elicits the biological or medicinal response in a tissue, system, or subject that is being sought by a researcher, veterinarian, medical doctor or other clinician, which includes one or more of the following disease prevention, *i.e.,* a subject who does not yet experience or display the pathology or symptomatology sufficient to definitively diagnose the disease; disease inhibition, *i.e.,* arresting or slowing further development of the pathology and/or symptomatology); and ameliorating the disease.

In one embodiment, the dosage of venlafaxine is about 75 mg to about 350 mg/day or about 75 mg to about 225 mg/day. In a further embodiment, the dosage of venlafaxine is about 75 mg to about 150 mg/day. When a DVS multiparticulate is utilized, the amount of the DVS multiparticulate will correspond to the dosage ranges for the venlafaxine free base. These may vary from patient to patient depending upon a patient's response rate, but generally will be at least 6:1 DVS to venlafaxine. Venlafaxine or another active agent delivered in a regimen with the multiparticulate of the invention may be formulated together with the multiparticulate of the invention, or delivered separately.

Formulations containing the present solid dispersions can be administered in daily doses ranging from 0.1 mg to 1000 mg of bazedoxifene acetate to a subject in need. In one embodiment, dose ranges vary from about 10 mg/day to about 600 mg/day, or from about 10 mg/day to about 60 mg/day. The dosing can be either in a single dose or two or more divided doses per day.

The active compounds described herein are typically combined with a pharmaceutical carrier or excipient (*e.g.,* pharmaceutically acceptable carriers and excipients) according to conventional pharmaceutical compounding technique to form a pharmaceutical composition or dosage form. Suitable pharmaceutically acceptable carriers and excipients include, but are not limited to, those described in Remington's, The Science and Practice of Pharmacy, (Gennaro, A. R, ed., 19th edition, 1995, Mack Pub. Co.). The phrase "pharmaceutically acceptable" refers to additives or compositions that are physiologically tolerable and do not typically produce an allergic or similar untoward reaction, such as gastric upset, dizziness and the like, when administered to an animal, such as a mammal (*e.g*., a human).

Oral solid pharmaceutical compositions may include, but are not limited to, starches, sugars, microcrystalline cellulose, diluents, granulating agents, lubricants, binders and disintegrating agents.

The following examples illustrate exemplary dosage forms of the products of the invention, and the use thereof. These examples are not a limitation of the present invention.

### EXAMPLE 1 - DVS-233 150mg /BZA 40mg BI-LAYER TABLETS

A tablet containing two layers having active compounds was prepared according to the following tables.

### A. Preparation

### Desvenlafaxine succinate 150mg granulation

| **Ingredients** | **Amount (mg)** |
|---|---|
| Desvenlafaxine succinate (DVS-233) | 227.62 |
| Hypomellose 2208 100,000 | 180.00 |
| Microcrystalline cellulose | 17.18 |
| Talc | 18.20 |
| Magnesium stearate | 7.00 |
| | |
| Total | 450.00 |

### Bazedoxifene acetate 40mg granulation

| **Ingredients** | **Amount (mg)** |
|---|---|
| Bazedoxifene acetate | 40.00 |
| Lactose | 134.20 |
| Microcrystalline cellulose | 130.20 |
| Pregelatinized starch | 56.00 |
| Sodium lauryl sulfate. | 6.00 |
| Sodium starch glycolate | 24.00 |
| Ascorbic acid | 6.00 |
| Silicon dioxide | 0.6 |
| Magnesium stearate | 3.00 |
| Water | Qs |
| | |
| Total | 400.00 |

The DVS-233 granulation was prepared via dry granulation using an Alexanderwerks roller compactor. The bazedoxifene granulation was prepared using a high shear granulator (Collette Gral) and dried in a fluid bed dryer. Bilayer tablets were compressed using a Carver press with 0.735" x 0.325" capsule shaped tooling.

### B. Stability

80 DVS / Bazedoxifene bilayered tablets (150 mg/40mg respectively) were stored at 25°C/60 relative humidity (RH) and 40°C/75 RH in a 50-mL high density polyethylene (HDPE) closed container for 2 weeks, 1 month and 3 months. The strengths of each active constituents and the dissolution rate in 0.2% Tween 80 / 10 mM Acetic acid at 37°C were monitored.

Strength (% Recovery) of BZA and ODV for different treatments is shown in the following table.

| | BZA 25°C/60 RH | BZA 40°C /75 RH | DVS 25°C /60 RH | DVS 40°C /75 RH |
|---|---|---|---|---|
| Initial | 96.40 | | 97.98 | |
| 2 weeks | 92.95 | 93.03 | 101.44 | 97.43 |
| 1 month | 100.20 | 100.21 | 102.36 | 103.65 |
| 3 months | 98.25 | 96.04 | 103.31 | 101.27 |

### Both BZA and DVS are stable in 25/60 and 40/75 for 3 months.

Dissolution of these bilayer tablets also showed that almost complete releases in 24 hours were achieved for both BZA and DVS. Where BZA gave a % release of more than 75% in 45 minutes and more than 93% release in 24 hours. DVS gave a slow release in the first 2 hours but it gave a complete release in 24 hours. Tablets core stay intact in the dissolution vessels after 24 hours and tablets that was treated at 40°C /75 RH for 3 months look more discolored (more faint-yellowish) than tablets of 25°C/60 RH treatments. These tablets were designed as an immediate release for BZA and a slow release for DVS and the dissolution pattern was consistent with this design.

### EXAMPLE 2 - DVS-233 multiparticulates/BZA Granulation Combination Capsule

DVS-233 multiparticulates were prepared as described in co-owned US Patent Publication No. US 2005/0175698 A1, published August 11, 2005, for *"Multiparticulate O-Desmethylvenlafaxine Salts and Uses Thereof"* (Diorio, et al).

### A. Desvenlafaxine succinate Multiparticulates

| **Ingredient** | **Grams/2000 grams** | **% wt/wt** |
|---|---|---|
| Desvenlafaxine succinate | 1400.0 | 70.0 |
| Microcrystalline cellulose | 600.0 | 30.0 |
| Water | Qs | qs |

The multiparticulate consists of a pellet core and non-functional seal coating and a functional second coating. The manufacturing of the multiparticulate core was as follows. The desvenlafaxine succinate (DVS-233) is combined with microcrystalline cellulose and granulated with water in a planetary mixer. Then using the Nica^{®} System the resulting wet mass is extruded through a 1.0mm screen. The DVS-233 extrudates are then transferred to the spheronizer and spun at approximately 700 rpm until spherical pellets are obtained (2-3 minutes).

The wet pellets are then dried in an Aeromatic Strea fluid bed dryer to a moisture level of 2-5%. The dried pellets are passed through a 18 mesh screen to remove larger oversize pellets. The pellets are now ready for the coating process.

### B. Coating

### 1. Seal Coat

| **Ingredient** | **Grams/500 grams** | **% wt/wt** |
|---|---|---|
| Opadry® Clear | 25.0 | 5 |
| Water | 475.0 | 95.0 |

The Aeromatic Strea fluid bed apparatus is fitted with a Wurster column and bottom spray nozzle system. Approximately 200 grams of the dried pellet cores are charged into the unit. The Opadry® seal coat is applied with a inlet temperature of approximately 60°C, a coating solution spray rate of 5-10 grams/minute, atomization pressure of 1-2 bar. The desired product temperature is 38°C-43°C. After approximately a 2% weight gain of the seal coat is achieved the ethylcellulose coat can be applied.

### 2. Ethylcellulose coat

| **Ingredient** | **Grams/ 500 grams** | **% wt/wt** |
|---|---|---|
| Surelease ® (aqueous ethylcellulose dispersion 25% solids) | 25.0 | 5 |
| Water | 475.0 | 95.0 |

The ethylcellulose is applied in a similar fashion as the seal coat to a weight gain of 3-4%. After the ethylcellulose coat is applied, the pellets are dried for an additional 5-10 minutes. They are removed and screened through an 18 mesh screen to remove agglomerates and oversized particles.

### C. DVS-233 multiparticulates 150mg/BZA 40mg Capsules

The coated *DVS-233* pellets are encapsulated to achieve a strength of 150mg of desvenlafaxine. The BZA granulation, as described above, is placed into the same hard gelatin capsule shell.

### EXAMPLE 3 - DVS-233 150mg /BZA 20mg Solid Dispersion Bi-layer tablets

### A. Desvenlafaxine succinate 150mg granulation

| **Ingredients** | **Amount (mg)** |
|---|---|
| Desvenlafaxine succinate (DVS-233) | 227.62 |
| Hypomellose 2208 100,000 | 180.00 |
| Microcrystalline cellulose | 17.18 |
| Talc | 18.20 |
| Magnesium stearate | 7.00 |
| Total | 450.00 |

### B. Bazedoxifene Solid Dispersion Blend

| **Ingredients** | **Amount (mg)** |
|---|---|
| Bazedoxifene:PVP solid dispersion | 44.64(A) |
| Microcrystalline cellulose | 54.26 |
| Croscarmellose sodium | 10.00 |
| Magnesium stearate | 1.10 |
| Total | 110.00 |

| | |
|---|---|
| A: equivalent to 20mg bazedoxifene | |

BZA acetate, Solid Dispersion with polyvinylpyrrolidone (PVP) at a ratio of 1:1 was prepared as described in co-owned US Patent Publication No. US 2005/0227966 A1 (published October 13, 2005), entitled "Bazedoxifene Acetate Formulations" (Shah, *et al*.).

In summary, to a solution of 3.00519 g of PVP K17 in 15 mL of ethanol, was added 3.00671 g of BZA with mixing. Another 60 mL of ethanol was added and the mixture was wormed to 65 °C for 5 minutes to get a clear yellow-brown solution. Solvents were evaporated under reduced pressure at room temperature to dryness. The yellow-brown solid was grinded with mortar and pestle to give yellow-creamy fine powder.

The DVS-233 granulation was prepared via dry granulation using an Alexanderwerks roller compactor. The bazedoxifene solid dispersion blend was prepared by direct blending using a mortar and pestle. Bilayer tablets were compressed using a Carver press with 0.450 inch square shaped tooling.

### EXAMPLE 4 - DVS-233 Multiparticulates 150 mg/BZA Solid Dispersion 20 mg Combination Capsule

The coated DVS-233 pellets described in Example 2 above are encapsulated to achieve a strength of 150mg of desvenlafaxine. The BZA solid dispersion blend as described in Example 3 above, is placed into the same hard gelatin capsule shell.

### EXAMPLE 5 - DVS-233 With BZA Overcoat

### A. DVS-233 100mg Sustained Release Tablet Cores

| **Ingredient** | **Amount per tablet** |
|---|---|
| desmethylvenlafaxine | 151.74 (100.0¹) |
| hypomellose 2208 100,000 cps | 170.0 |
| microcrystalline cellulose | 7.20 |
| Talc | 7.65 |
| magnesium stearate | 3.4 |

| | |
|---|---|
| ¹The DVS-233 potency of this tablet is equivalent to 100mg desmethylvenlafaxine free base. | |

### B. Bazedoxifene film-coat overcoat process

| **Ingredient** | **Amount per tablet (mg)** |
|---|---|
| DVS-233 100mg Tablet cores | 340.0 |
| | |
| *Coating Suspension* | |
| Opadry^{®} Clear Coat¹ | 42.5 |
| Bazedozifene acetate | 20.0 |

The Opadry Clear coat system contains hypomellose and is prepared at a concentration of 10% w/w.

The coating suspension is prepared by dissolving the Opadry Clear coat powder in the appropriate amount of water to obtain a solution that contains about 10% dissolved solids. The solution is mixed until a clear solution is obtained. Micronized bazedoxifene acetate is added to the Opadry solution slowly under vigorous mixing until a homogenous suspension is obtained. The Opadry/bazedoxifene suspension is sprayed onto the DVS-233 tablet cores under the following conditions using a Thomas LDCS 1.31 pan.

| | |
|---|---|
| Inlet Air Temperature | 60-80 °C |
| Exhaust Air Temperature | 38 - 42°C |
| Product Temperature | 42 - 48°C |
| Total Spray Rate | 6-10 g/min |
| Pan | 12-16 RPM |
| Atomizing Air | 15-25 psi |

### C. Bazedoxifene, sugar based, overcoat process

| **Ingredient** | **Amount per tablet (mg)** |
|---|---|
| DVS-233 100mg Tablet cores | 340.0 |
| | |
| *Coating Suspension* | |
| Sucrose | 78.0 |
| Bazedoxifene acetate | 20.0 |
| Hypomellose | 29.0 |
| Sucrose palmitate | 2.0 |
| Ascorbic acid | 2.0 |

A coating suspension is prepared by dissolving the sucrose, sucrose palmitate, hypomellose, and ascorbic acid in water. Micronized bazedoxifene acetate is added to the sucrose solution slowly under vigorous mixing until a homogenous suspension is obtained. The sucrose/bazedoxifene suspension is sprayed onto the DVS-233 tablet cores under the following conditions using a Thomas LDCS 1.31 pan.

| | |
|---|---|
| Inlet Air Temperature | 50-70°C |
| Exhaust Air Temperature | 35-45°C |
| Product Temperature | 42 - 48°C |
| Total Spray Rate | 6-10 g/min |
| Pan | 12-16 RPM |
| Atomizing Air | 15 - 25 psi |

A color and/or clear gloss coat may be applied after the sucrose/bazedoxifene suspension has been applied.

The present invention is not to be limited in scope by the specific embodiments described herein. Various modifications to these embodiments will be obvious to one of skill in the art from the description. Such modifications fall within the scope of the appended claims.

## Claims

1. An orally administrable combination product comprising as active compounds O-desmethylvenlafaxine succinate (DVS) in a sustained release formulation and bazedoxifene or a pharmaceutically acceptable salt thereof in an immediate release formulation.

2. The product according to claim 1, wherein the product comprises bazedoxifene acetate.

3. The product according to claims 1 or 2, which is a tablet.

4. The product according to claim 3, wherein the tablet is a multi-layer tablet, wherein the active compounds are located in separate layers.

5. The product according to any one of claims 1 to 4, wherein the bazedoxifene is provided in a coating layer over an O-desmethylvenlafaxine succinate core.

6. The product according to claim 5, wherein the bazedoxifene comprises about 20 mg of the product.

7. The product according to claims 1 or 2, which comprises a capsule.

8. The product according to any one of claims 1 to 7, which comprises 10 mg to 500 mg O-desmethylvenlafaxine succinate, based on the amount of O-desmethylvenlafaxine free base.

9. The product according to claim 8, which comprises 150 mg O-desmethylvenlafaxine succinate, based on the amount of O-desmethylvenlafaxine free base.

10. The product according to any one of claims 1 to 9, which comprises bazedoxifene or a pharmaceutically acceptable salt thereof in an amount of from 5 mg to 100 mg.

11. A tablet comprising a first sustained release layer comprising O-desmethylvenlafaxine succinate, hypomellose, microcrystalline cellulose, talc and magnesium stearate, and a second immediate release layer comprising bazedoxifene or a pharmaceutically acceptable salt thereof.

12. The tablet according to claim 11, wherein the second layer comprises bazedoxifene acetate, lactose, microcrystalline cellulose, and starch.

13. The tablet according to claims 11 or 12, wherein the first layer comprises a 10 mg to 500 mg granulation of O-desmethylvenlafaxine succinate comprising:
45 - 55 wt% O-desmethylvenlafaxine succinate;
35 - 45 wt % hypomellose;
3 - 4 wt % microcrystalline cellulose;
4 - 5 wt% talc; and
1 - 2 wt% magnesium stearate.

14. The tablet according to any one of claims 11 to 13, wherein the O-desmethylvenlafaxine succinate is about 50% and the hypomellose is about 40 wt%.

15. The tablet according to any one of claims 11 to 14, wherein the second layer comprises a 5 mg to 100 mg granulation of bazedoxifene acetate comprising:
10 wt% bazedoxifene acetate;
30 - 35 wt% lactose;
25 - 30 wt% microcrystalline cellulose;
12 -16 wt % pregelatized starch;
1 - 2 wt % sodium lauryl sulfate;
5 - 8 wt % sodium starch glycolate;
1 - 2 wt % ascorbic acid;
< 1 wt % silicon dioxide; and
< 1 wt % magnesium stearate.

16. The tablet according to claim 15, wherein the second layer comprises a 40 mg granulation of bazedoxifene acetate.

17. The tablet according to claim 11, wherein the second layer comprises a solid dispersion blend having an amount of bazedoxifene or a salt thereof equivalent to 5 mg to 100 mg bazedoxifene.

18. The tablet according to claim 17, wherein the bazedoxifene or salt thereof is a 20 mg solid dispersion blend.

19. The tablet according to claim 15, wherein the solid dispersion blend comprises:
about 40 wt% bazedoxifene:polyvinylpyrrolidone solid dispersion;
about 50 wt% microcrystalline cellulose;
about 10 wt% croscarmellose sodium; and
about 1 wt% magnesium stearate.

20. The tablet according to claim 11, wherein the bazedoxifene is provided in a coating layer in an amount of about 20 mg per tablet.

21. The tablet according to claim 20, wherein the coating layer comprises bazedoxifene acetate and hypomellose.

22. The product according to claim 7, wherein the capsule comprises O-desmethylvenlafaxine succinate in multiparticulates form and bazedoxifene or a pharmaceutically acceptable salt thereof.

23. The product according to claim 22, wherein the bazedoxifene or a pharmaceutically acceptable salt thereof is in the form of a solid dispersion.

24. The product according to claim 22, wherein the bazedoxifene or a pharmaceutically acceptable salt thereof is in the form of a granulation.

25. The product according to claim 22, wherein the bazedoxifene is a coating applied to the multiparticulates.

26. The product according to claim 22, wherein the multiparticulate comprises O-desmethylvenlafaxine succinate and microcrystalline cellulose.

27. Use of a combination product according to any one of claims 1 to 10 and 22 to 26 in the preparation of medicament useful in the treatment of hot flushes, osteoporosis, and/or vaginal atrophy.

28. Use of a combination product according to any one of claims 1 to 10 and 22 to 26 in the preparation of medicament useful in the treatment of disorders **characterized by** low circulating levels of estrogen.

29. Use of a combination product according to any one of claims 1 to 10 and 22 to 26 in the preparation of medicament useful in the treatment of depression, fibromyalgia, anxiety, stress urinary incontinence, and/or irritable bowel syndrome.

## Patentansprüche

1. Oral verabreichbares Kombinationsprodukt, umfassend als aktive Verbindungen O-Desmethylenvenlafaxinsuccinat (DVS) in einer Formulierung mit Langzeitfreisetzung und Bazedoxifen oder ein pharmazeutisch verträgliches Salz davon in einer Formulierung mit sofortiger Freisetzung.

2. Produkt nach Anspruch 1, wobei das Produkt Bazedoxifenacetat umfasst.

3. Produkt nach Anspruch 1 oder 2, welches eine Tablette ist.

4. Produkt nach Anspruch 3, wobei die Tablette eine Mehrschichttablette ist, wobei sich die aktiven Verbindungen in getrennten Schichten befinden.

5. Produkt nach einem der Ansprüche 1 bis 4, wobei das Bazedoxifen in einer Überzugsschicht über einem O-Desmethylvenlafaxinsuccinatkern vorgesehen ist.

6. Produkt nach Anspruch 5, wobei das Bazedoxifen etwa 20 mg des Produkts umfasst.

7. Produkt nach Anspruch 1 oder 2, welches eine Kapsel umfasst.

8. Produkt nach einem der Ansprüche 1 bis 7, welches 10 mg bis 500 mg O-Desmethylvenlafaxinsuccinat umfasst, bezogen auf die Menge der freien Base von O-Desmethylenvenlafaxin.

9. Produkt nach Anspruch 8, welches 150 mg O-Desmethylvenlafaxinsuccinat umfasst, bezogen auf die Menge der freien Base von O-Desmethylvenlafaxin.

10. Produkt nach einem der Ansprüche 1 bis 9, welches Bazedoxifen oder ein pharmazeutisch verträgliches Salz davon in einer Menge von 5 mg bis 100 mg umfasst.

11. Tablette, umfassend eine erste Schicht mit Langzeitfreisetzung, die O-Desmethylvenlafaxinsuccinat, Hypomellose, mikrokristalline Cellulose, Talk und Magnesiumstearat umfasst, und eine zweite Schicht mit sofortiger Freisetzung, die Bazedoxifen oder ein pharmazeutisch verträgliches Salz davon umfasst.

12. Tablette nach Anspruch 11, wobei die zweite Schicht Bazedoxifenacetat, Lactose, mikrokristalline Cellulose und Stärke umfasst.

13. Tablette nach Anspruch 11 oder 12, wobei die erste Schicht eine 10 mg bis 500 mg Granulierung von O-Desmethylvenlafaxinsuccinat umfasst, umfassend:
45 - 55 Gew.-% O-Desmethylvenlafaxinsuccinat;
35 - 45 Gew.-% Hypomellose;
3 - 4 Gew.-% mikrokristalline Cellulose;
4 - 5 Gew.-% Talk; und
1 - 2 Gew.-% Magnesiumstearat.

14. Tablette nach einem der Ansprüche 11 bis 13, wobei das O-Desmethylvenlafaxinsuccinat etwa 50% und die, Hypomellose etwa 40 Gew.-% betragen.

15. Tablette nach einem der Ansprüche 11 bis 14, wobei die zweite Schicht eine 5 mg bis 100 mg Granulierung von Bazedoxifenacetat umfasst, umfassend:
10 Gew.-% Bazedoxifenacetat;
30 - 35 Gew.-% Lactose;
25 - 30 Gew.-% mikrokristalline Cellulose;
12 - 16 Gew.-% vorgelatinisierte Stärke;
1 - 2 Gew.-% Natriumlaurylsulfat;
5 - 8 Gew.-% Natriumstärkeglycolat;
1 - 2 Gew.-% Ascorbinsäure;
< 1 Gew.-% Siliziumdioxid; und
< 1 Gew.-% Magnesiumstearat.

16. Tablette nach Anspruch 15, wobei die zweite Schicht eine 40 mg Granulierung von Bazedoxifenacetat umfasst.

17. Tablette nach Anspruch 11, wobei die zweite Schicht eine Feststoffdispersionsmischung mit einer Menge von Bazedoxifen oder einem Salz davon äquivalent zu 5 mg bis 100 mg Bazedoxifen umfasst.

18. Tablette nach Anspruch 17, wobei das Bazedoxifen oder das Salz davon eine 20 mg Feststoffdispersionsmischung ist.

19. Tablette nach Anspruch 15, wobei die Feststoffdispersionsmischung umfasst:
etwa 40 Gew.-% Feststoffdispersion Bazedoxifen:Polyvinylpyrrolidon;
etwa 50 Gew.-% mikrokristalline Cellulose;
etwa 10 Gew.-% Croscarmellose-Natrium; und etwa 1 Gew.-% Magnesiumstearat.

20. Tablette nach Anspruch 11, wobei das Bazedoxifen in einer Überzugsschicht in einer Menge von etwa 20 mg pro Tablette vorgesehen ist.

21. Tablette nach Anspruch 20, wobei die Überzugsschicht Bazedoxifenacetat und Hypomellose umfasst.

22. Produkt nach Anspruch 7, wobei die Kapsel O-Desmethylvenlafaxinsuccinat in multipartikulärer Form und Bazedoxifen oder ein pharmazeutisch verträgliches Salz davon umfasst.

23. Produkt nach Anspruch 22, wobei das Bazedoxifen oder ein pharmazeutisch verträgliches Salz davon in Form einer Feststoffdispersion vorliegt.

24. Produkt nach Anspruch 22, wobei das Bazedoxifen oder ein pharmazeutisch verträgliches Salz davon in Form einer Granulierung vorliegt.

25. Produkt nach Anspruch 22, wobei das Bazedoxifen ein Überzug ist, der auf die Multipartikel aufgebracht wird.

26. Produkt nach Anspruch 22, wobei die Multipartikel O-Desmethylvenlafaxinsuccinat und mikrokristalline Cellulose umfassen.

27. Verwendung eines Kombinationsproduktes nach einem der Ansprüche 1 bis 10 und 22 bis 26 bei der Herstellung eines Medikaments, das zur Behandlung von Hitzewallungen, Osteoporose und/oder Scheidenatrophie nützlich ist.

28. Verwendung eines Kombinationsproduktes nach einem der Ansprüche 1 bis 10 und 22 bis 26 bei der Herstellung eines Medikaments, das zur Behandlung von Erkrankungen nützlich ist, die durch einen niedrigen Östrogenspiegel gekennzeichnet sind.

29. Verwendung eines Kombinationsproduktes nach einem der Ansprüche 1 bis 10 und 22 bis 26 bei der Herstellung eines Medikaments, das zur Behandlung von Depressionen, Fibromyalgie, Angstzuständen, stressbedingter Harninkontinenz und/oder irritablem spastischem Kolon nützlich ist.

## Revendications

1. Produit de combinaison administrable par voie orale, comprenant, à titre de composés actifs, du succinate de O-desméthylvenlafaxine (DVS) dans une formulation à libération soutenue et du bazédoxifène ou un de ses sels pharmaceutiquement acceptables dans une formulation à libération immédiate.

2. Produit selon la revendication 1, dans lequel le produit comprend de l'acétate de bazédoxifène.

3. Produit selon la revendication 1 ou 2, à savoir un comprimé.

4. Produit selon la revendication 3, dans lequel le comprimé est un comprimé multicouche, les composés actifs étant disposés dans des couches séparées.

5. Produit selon l'une quelconque des revendications 1 à 4, le bazédoxifène est fourni dans une couche d'enrobage par-dessus un noyau de succinate de O-desméthylvenlafaxine.

6. Produit selon la revendication 5, dans lequel le bazédoxifène comprend environ 20 mg du produit.

7. Produit selon la revendication 1 ou 2, qui comprend une capsule.

8. Produit selon l'une quelconque des revendications 1 à 7, qui comprend, à concurrence de 10 mg à 500 mg, du succinate de O-desméthylvenlafaxine, en se basant sur la quantité de la base libre de O-desméthylvenlafaxine.

9. Produit selon la revendication 8, qui comprend 150 mg de succinate de O-desméthylvenlafaxine basés sur la quantité de la base libre de O-desméthylvenlafaxine.

10. Produit selon l'une quelconque des revendications 1 à 9, qui comprend du bazédoxifène ou un de ses sels pharmaceutiquement acceptables en une quantité de 5 mg à 100 mg.

11. Comprimé comprenant une première couche à libération soutenue comprenant du succinate de O-desméthylvenlafaxine, de l'hypomellose, de la cellulose microcristalline, du talc et du stéarate de magnésium, et une deuxième couche à libération immédiate comprenant du bazédoxifène ou un de ses sels pharmaceutiquement acceptables.

12. Comprimé selon la revendication 11, dans lequel la deuxième couche comprend de l'acétate de bazédoxifène, du lactose, de la cellulose microcristalline et de l'amidon.

13. Comprimé selon la revendication 11 ou 12, dans lequel la première couche comprend, à concurrence de 10 mg à 500 mg un produit de granulation de succinate de O-desméthylvenlafaxine comprenant :
à concurrence de 45 à 55 % en poids, du succinate de O-desméthylvenlafaxine ;
à concurrence de 35 à 45 % en poids, de l'hypomellose ;
à concurrence de 3 à 4 % en poids, de la cellulose microcristalline ;
à concurrence de 4 à 5 % en poids, du talc ; et
à concurrence de 1 à 2 % en poids, du stéarate de magnésium.

14. Comprimé selon l'une quelconque des revendications 11 à 13, dans lequel le succinate de O-desméthylvenlafaxine représente environ 50 % et l'hypomellose représente environ 40 % en poids.

15. Comprimé selon l'une quelconque des revendications 11 à 14, dans lequel la deuxième couche comprend, à concurrence de 5 mg à 100 mg, un produit de granulation d'acétate de bazédoxifène comprenant :
à concurrence de 10 % en poids, de l'acétate de bazédoxifène ;
à concurrence de 30 à 35 % en poids, du lactose ;
à concurrence de 25 à 30 % en poids, de la cellulose microcristalline ;
à concurrence de 12 à 16 % en poids, de l'amidon prégélatinisé ;
à concurrence de 1 à 2 % en poids, du laurylsulfate de sodium ;
à concurrence de 5 à 8 % en poids, du glycolate d'amidon de sodium ;
à concurrence de 1 à 2 % en poids, de l'acide ascorbique ;
à concurrence de moins de 1 % en poids, du dioxyde de silicium ; et
à concurrence de moins de 1 % en poids, du stéarate de magnésium.

16. Comprimé selon la revendication 15, dans lequel la deuxième couche comprend, à concurrence de 40 mg, un produit de granulation d'acétate de bazédoxifène.

17. Comprimé selon la revendication 11, dans lequel la deuxième couche comprend un mélange de dispersion solide possédant une quantité de bazédoxifène ou d'un de ses sels, équivalente à 5 mg à 100 mg de bazédoxifène.

18. Comprimé selon la revendication 17, dans lequel le bazédoxifène ou son sel représente un mélange de dispersion solide à concurrence de 20 mg.

19. Comprimé selon la revendication 15, dans lequel le mélange de dispersion solide comprend :
à concurrence d'environ 40 % en poids, une dispersion solide de bazédoxifène:polyvinylpyrrolidone ;
à concurrence d'environ 50 % en poids, de la cellulose microcristalline ;
à concurrence d'environ 10 % en poids, du croscarmellose sodique ; et
à concurrence d'environ 1% en poids, du stéarate de magnésium.

20. Comprimé selon la revendication 11, dans lequel le bazédoxifène est fourni dans une couche d'enrobage en une quantité d'environ 20 mg par comprimé.

21. Comprimé selon la revendication 20, dans lequel la couche d'enrobage comprend de l'acétate de bazédoxifène et de l'hypomellose.

22. Produit selon la revendication 7, dans lequel la capsule comprend du succinate de O-desméthylvenlafaxine sous forme de particules multiples et du bazédoxifène ou un de ses sels pharmaceutiquement acceptables.

23. Produit selon la revendication 22, dans lequel le bazédoxifène ou un de ses sels pharmaceutiquement acceptables se présente sous la forme d'une dispersion solide.

24. Produit selon la revendication 22, dans lequel le bazédoxifène ou un de ses sels pharmaceutiquement acceptables se présente sous la forme d'un produit de granulation.

25. Produit selon la revendication 22, dans lequel le bazédoxifène est une couche d'enrobage appliquée sur les particules multiples.

26. Produit selon la revendication 22, dans lequel les particules multiples comprennent du succinate de O-desméthylvenlafaxine et de la cellulose microcristalline.

27. Utilisation d'un produit de combinaison selon l'une quelconque des revendications 1 à 10 et 22 à 26, dans la préparation d'un médicament utile dans le traitement des bouffées de chaleur, de l'ostéoporose et/ou de l'atrophie vaginale.

28. Utilisation d'un produit de combinaison selon l'une quelconque des revendications 1 à 10 et 22 à 26, dans la préparation d'un médicament utile dans le traitement de troubles **caractérisés par** de faibles taux d'oestrogènes en circulation.

29. Utilisation d'un produit de combinaison selon l'une quelconque des revendications 1 à 10 et 22 à 26, dans la préparation d'un médicament utile dans le traitement de la dépression, de la fibromyalgie, de l'anxiété, de l'incontinence à l'effort et/ou du syndrome du côlon irritable.
